(19) Europäisches Patentamt
European Patent Office
Office européen des brevets

(11) Publication number: **0 428 268 A2**

(12) **EUROPEAN PATENT APPLICATION**

(21) Application number: **90311194.6**

(22) Date of filing: **12.10.90**

(51) Int. Cl.5: **C07D 213/85**, C07D 213/82, C07D 213/64, A61K 31/44

The title of the invention has been amended (Guidelines for Examination in the EPO, A-III, 7.3).

(30) Priority: **13.10.89 GB 8923131**

(43) Date of publication of application: **22.05.91 Bulletin 91/21**

(84) Designated Contracting States: **AT BE CH DE DK ES FR GB GR IT LI LU NL SE**

(71) Applicant: **SMITH KLINE & FRENCH LABORATORIES LIMITED**
**Mundells**
**Welwyn Garden City Hertfordshire, AL7 1EY(GB)**

(72) Inventor: **Coates, William John**
**Smith Kline & French Res. Ltd., The Frythe, Welwyn**
**Hertfordshire, AL6 9AR(GB)**
Inventor: **Flynn, Sean Thomas**
**Smith Kline & French Res. Ltd., The Frythe, Welwyn**
**Hertfordshire, AL6 9AR(GB)**

(74) Representative: **Waters, David Martin, Dr. et al**
**Corporate Patents, Smithkline Beecham,2 Mundells**
**Welwyn Garden City Hertfordshire AL7 1EY(GB)**

(54) **Pharmaceutical phenylpyridone derivatives.**

(57) Compounds of the formula (1) :

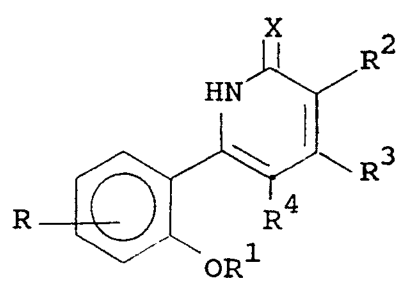

(1)

and pharmaceutically acceptable salts are described, wherein X is O or S; $R^1$ is $C_{1-6}$alkyl, $C_{2-6}$alkenyl, $C_{3-5}$cycloalkyl$C_{1-4}$alkyl, or $C_{1-4}$alkyl substituted by 1 to 3 fluoro groups; $R^2$ is hydrogen, -CN, $-CONR^5R^6$, $-CO_2R^7$, 5-tetrazolyl, $-NO_2$, $-NH_2$ or $-NHCOR^8$ wherein $R^5$ to $R^8$ are independently hydrogen or $C_{1-4}$alkyl; $R^3$ is hydrogen or $C_{1-4}$alkyl; $R^4$ is hydrogen or $C_{1-4}$alkyl; and R is halo, $C_{1-4}$alkyl, $C_{1-4}$alkoxy, cyano, $-CONR^9R^{10}$, $-CO_2R^{11}$, $-S(0)_nC_{1-4}$alkyl, $-NO_2$, $-NH_2$, $-NHCOR^{12}$, or $-SO_2NR^{13}R^{14}$ wherein n is 0, 1 or 2 and $R^9$ to $R^{14}$ are independently hydrogen or $C_{1-4}$alkyl; with the proviso that $R^1$ is not methyl when $R^2$ is $-CO_2H$, $-CO_2CH_2CH_3$ or -CN, X is 0, $R^3$ is hydrogen, $R^4$ is hydrogen or methyl and R is 6-methoxy.

These compounds have bronchodilator, anti-allergic, vasodilator and anti-inflammatory activities. Pharmaceutical compositions are described as are methods of use. Processes for the preparation of the compounds of the formula (1) are described.

EP 0 428 268 A2

## CHEMICAL COMPOUNDS

The present invention relates to phenylpyridone derivatives. This invention further relates to processes for their preparation, their use as therapeutic agents and to pharmaceutical compositions containing them. The compounds of this invention are inhibitors of a calmodulin insensitive cyclic GMP phosphodiesterase and are of use in combatting such conditions where such inhibition is thought to be beneficial. The prime activities of the compounds of this invention are bronchodilatation and anti-allergic activity. The compounds of this invention are therefore of use in combatting chronic reversible obstructive lung diseases such as asthma and bronchitis and in combatting allergic diseases such as allergic asthma, allergic rhinitis, urticaria and irritable bowel syndrome. Furthermore the compounds of this invention are vasodilators and are therefore of value in combatting angina, hypertension and congestive heart failure. The compounds of this invention also exhibit anti-inflammatory activity.

GB patent application 2070606 discloses as cardiotonic agents compounds of the formula (A):

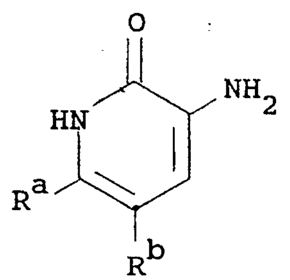

(A)

wherein $R^a$ is inter alia phenyl optionally substituted by $C_{1-4}$alkoxy and $R^b$ is inter alia hydrogen or $C_{1-4}$alkyl. None of the compounds of the present invention are specifically disclosed.

US Patent 3703582 discloses compounds of the formula (B):

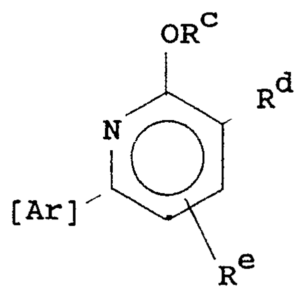

(B)

wherein [Ar] is inter alia optionally substituted phenyl, $R^c$ is inter alia hydrogen, $R^d$ is carboxy, alkoxycarbonyl, carbamoyl, or alkanoylamino and $R^e$ is inter alia hydrogen or alkyl. The compounds are described as having anti-inflammatory, anti-pyretic, analgesic, diuretic, anti-fibrinolytic and hypoglycemic activities or as being intermediates in the preparation thereof. Compounds of the formula (B) that are specifically disclosed include compounds wherein [Ar] is 2,6-dimethoxyphenyl, $R^c$ is hydrogen, $R^d$ is -CN, $-CO_2H$ or $-CO_2CH_2CH_3$ and $R^e$ is hydrogen or 5-methyl. None of the compound of the present invention are specifically disclosed.

US Patent 4530842 discloses compounds of the formula (C) :

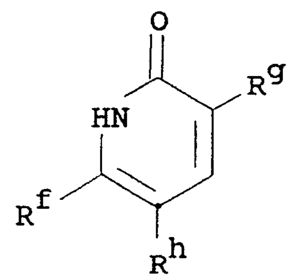

(C)

wherein $R^f$ is inter alia phenyl disubstituted by a group $-(CH_2)_mX$ or $-(CH_2)_nY$ wherein m is 0 to 4, n is 1 to 4, X is cyano, carboxy, alkoxycarbonyl, carbamoyl or alkylsulphonyl and Y is hydroxy, alkoxy or halogen, and by alkyl, alkoxy or halo, $R^h$ is hydrogen or alkyl and $R^g$ is cyano, carbamoyl or amino. The compounds are described as having cardiotonic activity. None of the compounds of the present invention are specifically disclosed.

According to the present invention there is provided compounds of the formula (1) :

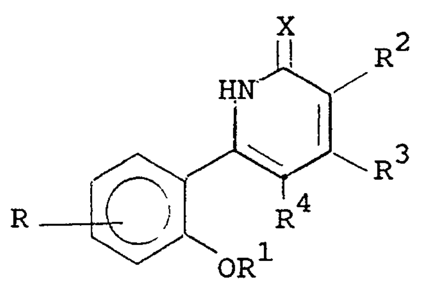

(1)

and pharmaceutically acceptable salts thereof, wherein

X is O or S;

$R^1$ is $C_{1-6}$alkyl, $C_{2-6}$alkenyl, $C_{3-5}$cycloalkyl$C_{1-4}$alkyl, or $C_{1-4}$alkyl substituted by 1 to 3 fluoro groups;

$R^2$ is hydrogen, -CN, $-CONR^5R^6$, $-CO_2R^7$,5-tetrazolyl, $-NO_2$, $-NH_2$ or $-NHCOR^8$ wherein $R^5$ to $R^8$ are independently hydrogen or $C_{1-4}$alkyl;

$R^3$ is hydrogen or $C_{1-4}$alkyl;

$R^4$ is hydrogen or $C_{1-4}$alkyl; and

R is halo, $C_{1-4}$alkyl, $C_{1-4}$alkoxy, cyano, $-CONR^9R^{10}$, $-CO_2R^{11}$, $-S(0)_nC_{1-4}$alkyl, $-NO_2$, $-NH_2$, $-NHCOR^{12}$, or $-SO_2NR^{13}R^{14}$ wherein n is 0, 1 or 2 and $R^9$ to $R^{14}$ are independently hydrogen or $C_{1-4}$alkyl;

with the proviso that $R^1$ is not methyl when $R^2$ is $-CO_2H$, $-CO_2CH_2CH_3$ or -CN, X is 0, $R^3$ is hydrogen, $R^4$ is hydrogen or methyl and R is 6-methoxy.

Suitably X is S. Preferably X is O.

Suitably $R^1$ is $C_{2-6}$alkyl for example ethyl, n-propyl, isopropyl, butyl, isobutyl, pentyl or hexyl.

Suitably $R^1$ is $C_{3-5}$alkenyl for example allyl, butenyl or pentenyl.

Suitably $R^1$ is cyclopropylmethyl.

An example of $C_{1-4}$alkyl substituted by 1 to 3 fluoro groups is $-CH_2CF_3$.

Preferably $R^1$ is n-propyl.

Suitably $R^2$ is hydrogen, -CN, 5-tetrazolyl or $-NO_2$.

Suitably $R^2$ is $-CONR^5R^6$ wherein $-NR^5R^6$ is amino.

Suitably $R^2$ is $-CO_2R^7$ wherein $R^7$ is methyl.

Suitably $R^3$ is hydrogen or methyl.

Suitably $R^4$ is hydrogen or methyl.

Suitably R is halo for example fluoro, chloro, bromo or iodo.

Suitably R is $C_{1-4}$alkyl or $C_{1-4}$alkoxy for example methyl, ethyl, methoxy, ethoxy or propoxy.

Suitably R is cyano, $-CONR^9R^{10}$ for example $-CONH_2$, $-CONHCH_3$ or $-CON(CH_3)_2$ or $-CO_2R^{11}$ wherein $R^{11}$ is $C_{1-4}$alkyl.

Suitably R is $-NO_2$, $-NH_2$ or $-NHCOR^{12}$ wherein $R^{12}$ is $C_{1-4}$alkyl.

Suitably R is $-S(0)_nC_{1-4}$alkyl for example methylthio, ethylthio, methylsulphinyl, ethylsulphinyl, methylsulphonyl or ethylsulphonyl.

Suitably R is $SO_2NR^{13}R^{14}$ for example sulphamoyl, N-methylsulphamoyl or N,N-dimethylsulphamoyl.

Specific compounds of this invention are :

3-cyano-6-(2-methoxy-4-methylthiophenyl)-2(1H)-pyridinone,
3-cyano-6-(4-methylthio-2-propoxyphenyl)-2(1H)-pyridinone,
1,2-dihydro-6-(4-methylthio-2-propoxyphenyl)-2-oxo-3-pyridine carboxamide,
3-cyano-6-(2-methoxy-4-methylsulphinylphenyl)-2(1H)-pyridinone,
3-cyano-6-(4-methylsulphinyl-2-propoxyphenyl)-2(1H)-pyridinone,
3-cyano-6-(4-methylsulphonyl-2-propoxyphenyl)-2((1H)-pyridinone,
3-cyano-6-(2-methoxy-4-methylsulphonylphenyl)-2(1H)-pyridinone,
3-cyano-6-(5-fluoro-2-propoxyphenyl)-2(1H)-pyridinone,
1,2-dihydro-6-(5-fluoro-2-propoxyphenyl)-2-oxo-3-pyridine carboxamide,
3-cyano-6-(4-methoxy-2-propoxyphenyl)-2(1H)-pyridinone,
1,2-dihydro-6-(4-methoxy-2-propoxyphenyl)-2-oxo-3-pyridine carboxamide,
3-cyano-6-(5-methoxy-2-propoxyphenyl)-2(1H)-pyridinone,
1,2-dihydro-6-(5-methoxy-2-propoxyphenyl)-2-oxo-3-pyridine carboxamide,
3-cyano-6-(5-cyano-2-propoxyphenyl)-2(1H)-pyridinone,
3-(3-carboxamido-1,2-dihydro-2-oxo-6-pyridinyl)-4-propoxybenzamide,
methyl 3-(3-cyano-1,2-dihydro-(2-oxo-6-pyridinyl)-4-propoxybenzoate,
3-(3-cyano-1,2-dihydro-2-oxo-6-pyridinyl)-4-propoxybenzamide,
N-methyl-3-(3-cyano-1,2-dihydro-2-oxo-6-pyridinyl)-4-propoxybenzamide,
N-methyl 3-(3-carboxamido-1,2-dihydro-2-oxo-6-pyridinyl)-4-propoxybenzamide,
N,N-dimethyl-3-(3-cyano-1,2-dihydro-2-oxo-6-pyridinyl)-4-propoxybenzamide,
N,N-dimethyl 3-(3-carboxamido-1,2-dihydro-2-oxo-6-pyridinyl)-4-propoxybenzamide,
4-(3-cyano-1,2-dihydro-2-oxo-6-pyridinyl)-3-propoxybenzonitrile,
4-(3-carboxamido-1,2-dihydro-2-oxo-6-pyridinyl)-3-propoxybenzamide,
3-cyano-6-(5-methylthio-2-propoxyphenyl)-2(1H)pyridinone,
3-(3-cyano-1,2-dihydro-2-oxo-6-pyridinyl)-4-propoxy-N,N-dimethylbenzenesulphonamide,
3-(3-carboxamido-1,2-dihydro-2-oxo-6-pyridinyl)-4-propoxy-N,N-dimethylbenzenesulphonamide,
6-(2-cyclopropylmethoxy-5-flourophenyl)-1,2-dihydro-2-oxo-pyridine-3-carboxamide,
6-(5-fluoro-2-(2-methylpropoxy)phenyl)-1,2-dihydro-2-oxo-pyridine-3-carboxamide,
3-cyano-6-(5-nitro-2-propoxyphenyl)-2(1H)-pyridinone,
1,2-dihydro-6-(5-nitro-2-propoxyphenyl)-2-oxo-3-pyridinone carboxamide,
3-cyano-6-(5-amino-2-propoxyphenyl)-2(1H)-pyridinone,
1,2-dihydro-6-(5-amino-2-propoxyphenyl)-2-oxo-3-pyridinone carboxamide,
3-cyano-6-(5-acetamido-2-propoxyphenyl)-2(1H)-pyridinone or
1,2-dihydro-6-(5-acetamido-2-propoxyphenyl)-2-oxo-3-pyridine carboxamide,
or pharmaceutically acceptable salts thereof.

This invention covers all tautomeric and optical isomeric forms of compounds of formula (1).

Compounds of the formula (1) may form pharmaceutically acceptable salts with metal ions, such as alkali metals for example sodium and potassium, or with an ammonium ion.

In order to use a compound of the formula (1) or a pharmaceutically acceptable salt thereof for the treatment of humans and other mammals it is normally formulated in accordance with standard pharmaceutical practice as a pharmaceutical composition.

Compounds of formula (1) and their pharmaceutically acceptable salts may be administered in standard manner for the treatment of the indicated diseases, for example orally, sublingually, parenterally, transdermally, rectally, via inhalation or via buccal administration.

Compounds of formula (1) and their pharmaceutically acceptable salts which are active when given orally or via buccal administration can be formulated appropriately in dosage forms such as liquids, syrups, tablets, capsules and lozenges. An oral liquid formulation will generally consist of a suspension or solution of the compound or salt in a liquid carrier for example, ethanol, glycerine or water with a flavouring or colouring agent. Where the composition is in the form of a tablet, any pharmaceutical carrier routinely used for preparing solid formulations may be used. Examples of such carriers include starch, celluloses, lactose, sucrose and magnesium stearate. Where the composition is in the form of a capsule, any routine encapsulation process may be suitable, for example using the aforementioned carriers in a hard gelatin capsule shell. Where the composition is in the form of a soft gelatin shell capsule, any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile

aqueous or non-aqueous carrier optionally containing a parenterally acceptable oil or solubilising agent, for example polyethylene glycol, polyvinylpyrrolidone, 2-pyrrolidone, cyclodextrin, lecithin, arachis oil or sesame oil.

A typical suppository formulation comprises a compound of formula (1) or a pharmaceutically acceptable salt thereof which is active when administered in this way, with a binding and/or lubricating agent, for example polymeric glycols, gelatins, cocoa-butter or other low melting vegetable waxes or fats or their synthetic analogues.

Typical transdermal formulations comprise a conventional aqueous or non-aqueous vehicle, for example a cream, ointment, lotion or paste cr are in the form of a medicated plaster, patch or membrane.

Typical compositions for inhalation are in the form of a solution, suspension or emulsion that may be administered in the form of an aerosol using a conventional propellant such as dichlorodifluoromethane or trichlorofluoromethane, or are in the form of a powder for insufflation.

Preferably the composition is in unit dosage form, for example a tablet, capsule or metered aerosol dose, so that the patient may administer to himself a single dose.

Each dosage unit for oral administration contains suitably from 0.001 mg/Kg to 30 mg/Kg, and preferably from 0.005 mg/Kg to 15 mg/Kg, and each dosage unit for parenteral administration contains suitably from 0.001 mg/Kg to 10 mg/Kg, of a compound of formula (1) or a pharmaceutically acceptable salt thereof calculated as the free base.

The daily dosage regimen for oral administration is suitably about 0.001 mg/Kg to 120 mg/Kg, of a compound of formula (1) or a pharmaceutically acceptable salt thereof calculated as the free base. The daily dosage regimen for parenteral administration is suitably about 0.001 mg/Kg to 40 mg/Kg, for example about 0.005 mg/Kg to 10 mg/Kg, of a compound of the formula (I) or a pharmaceutically acceptable salt thereof calculated as the free base. The active ingredient may be administered as required, for example from 1 to 8 times a day or by infusion. The compositions of the invention are bronchodilators and are useful in chronic reversible obstructive lung disease for example asthma and bronchitis. Some of the compositions of the present invention have anti-allergic activity and are therefore useful in combatting allergic diseases such as allergic asthma, allergic rhinitis, urticaria and irritable bowel syndrome. The compositions of the present invention have vasodilator activity and are of use in the treatment of angina, hypertension and congestive heart failure. Such conditions can be treated by administration orally, sublingually, topically, rectally, parenterally or by inhalation. For administration by inhalation dosages are controlled by a valve, are administered as required and for an adult are conveniently in the range 0.1-5.0 mg of a compound of the formula (1) or a pharmaceutically acceptable salt thereof.

The compounds of this invention may be co-administered with other pharmaceutically active compounds, for example in combination, concurrently or sequentially. Conveniently the compounds of this invention and the other active compound or compounds are formulated in a pharmaceutical composition. Examples of compounds which may be included in pharmaceutical compositions with the compounds of the formula (1) are bronchodilators such as sympathomimetic amines for example isoprenaline, isoetharine, salbutamol, phenylephrine and ephedrine or xanthine derivatives for example theophylline and aminophylline, anti-allergic agents for example disodium cromoglycate, histamine $H_1$- antagonists, vasodilators for example hydralazine, angiotensin converting enzyme inhibitors for example captopril, anti-anginal agents for example isosorbide nitrate, glyceryl trinitrate and pentaerythritol tetranitrate, anti-arrhythmic agents for example quinidine, procainamide and lignocaine, calcium antagonists for example verapamil and nifedipine, diuretics such as thiazides and related compounds for example bendrofluazide, chlorothiazide, chlorothalidone, hydrochlorothiazide, and other diuretics for example frusemide and triamterene, and sedatives for example nitrazepam, flurazepam and diazepam.

In another aspect the present invention provides a process for the preparation of compounds of the formula (1) or pharmaceutically acceptable salts thereof, which process comprises :

a) reacting a compound of the formula (2) :

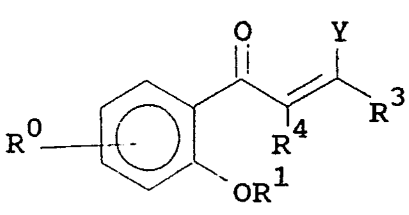

(2)

wherein $R^1$, $R^3$ and $R^4$ are as hereinbefore defined, $R^0$ is a group R as hereinbefore defined or a precursor thereof and Y is a displaceable group,
with a compound of the formula (3) :

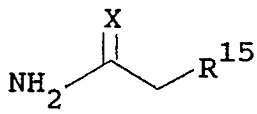

(3)

wherein X is as hereinbefore defined and $R^{15}$ is a group $R^2$ as hereinbefore defined or a precursor thereof;
b) reacting a compound of the formula (4) :

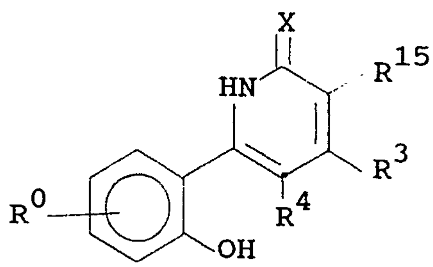

(4)

wherein $R^0$, X, $R^3$, $R^4$ and $R^{15}$ are as hereinbefore defined,
with a reagent that provides an $R^1$ group, and thereafter where necessary :
° converting a group $R^{15}$ to a group $R^2$;
° converting a group $R^0$ to a group R,
° optionally forming a pharmaceutically acceptable salt.

Suitably Y is hydroxy or a derivative thereof for example Y is protected hydroxy such as silyloxy, an acid residue (for example $C_{1-6}$alkanoyloxy) or an ether residue (for example methoxy or ethoxy). Alternatively Y is a secondary amino group, for example di-$C_{1-6}$alkylamino such as dimethylamino or a cyclic amino group such as piperidino, pyrrolidino or morpholino. Preferably Y is hydroxy or dimethylamino.

Suitably an alkali metal (e.g. sodium) salt of a compound of the formula (2) wherein Y is hydroxy is treated with a compound of the formula (3) under mildly alkaline aqueous conditions, for example in water in the presence of piperidine and glacial acetic acid, at an elevated temperature e.g. 30-200° C, preferably at the reflux temperature of the reaction mixture.

Alternatively a compound of the formula (2) wherein Y is a secondary amino group, for example dimethylamino, is treated with a compound of the formula (3) in a suitable solvent such as dimethylformamide, a $C_{1-4}$alkanol or pyridine at an elevated temperature e.g. 30-200° C, preferably at the reflux temperature of the reaction mixture optionally in the presence of a base such as pyridine or an alkali metal alkoxide, e.g. sodium methoxide.

Suitably a compound of the formula (4) is treated with a reagent that provides an $R^1$ group in an organic solvent such as acetone, dimethylformamide, dimethylsulphoxide or a $C_{1-4}$alkanol such as methanol or ethanol in the presence of a base such as potassium carbonate, potassium hydroxide, sodium hydroxide, potassium tert-butoxide or sodium hydride at ambient or elevated temperature e.g 30-100° C. An example of a reagent that provides an $R^1$ group is a compound of the formula $R^1Z$ wherein $R^1$ is as hereinbefore defined and Z is a leaving group. Suitably Z is a halo (such as chloro or bromo), tosyl, mesyl or $OSO_3R^1$ group wherein $R^1$ is as hereinbefore defined.

An example of $R^{15}$ being a precursor of the group $R^2$ is when $R^{15}$ is a cyano group. Such a group can be hydrolysed to a carboxamido group in conventional manner, for example by treatment with concentrated sulphuric acid at moderate temperature or by treatment with aqueous hydrogen peroxide and potassium hydroxide or by treatment with manganese dioxide on silica gel. The preparation as hereinbefore described of a compound of the formula (1) wherein $R^2$ is cyano may be accompanied by the formation of the corresponding compound wherein $R^2$ is carboxamido. Suitably the product mixture is separated in conventional manner to yield the corresponding compounds wherein $R^2$ is cyano or carboxamido.

A compound of the formula (1) wherein $R^2$ is a cyano group can be converted to the corresponding

compound wherein $R^2$ is carboxy by hydrolysis for example using aqueous potassium hydroxide at elevated temperature, for example at the reflux temperature of the the reaction mixture or in a pressure vessel at 100-160° C.

A compound of the formula (1) wherein $R^2$ is a cyano group can be converted to a compound of formula (1) wherein $R^2$ is 5-tetrazolyl in conventional manner, for example by treatment with a suitable azide salt such as ammonium, sodium, potassium or aluminium azide in a suitable organic solvent such as dimethylformamide, dimethylsulphoxide, N-methylpyrrolidin-2-one or tetrahydrofuran at an elevated temperature e.g. 40-200° C, preferably at the reflux temperature of the the reaction mixture.

A compound of the formula (1) wherein $R^2$ is carboxy can be esterified to a compound of formula (1) wherein $R^2$ is $-CO_2R^7$ by treatment with $R^7OH$ wherein $R^7$ is as hereinbefore defined in the presence of an acid catalyst.

A compound of the formula (1) wherein $R^2$ is $-CONR^5R^6$ can be prepared by reacting a compound of the formula (1) wherein $R^2$ is $-CO_2R^7$ with an amine $HNR^5R^6$ wherein $R^5$ and $R^6$ are as hereinbefore defined.

A compound of the formula (1) wherein $R^2$ is hydrogen can be prepared by decarboxylating a compound of the formula (1) wherein $R^2$ is carboxy, for example by heating at elevated temperature in the absence of a solvent or in the presence of a solvent such as quinoline.

Another example of $R^{15}$ being a precursor to the group $R^2$ is when $R^{15}$ is a nitro group. Such a group can be reduced to an amino group in conventional manner, for example via catalytic hydrogenation, for example using hydrogen gas or catalytic transfer hydrogenation.

Compounds of the formula (1) wherein $R^2$ is amino can be converted to compounds of the formula (1) wherein $R^2$ is $-NHCOR^8$ by conventional methods of acylation, for example using an acid halide, an acid anhydride or an activated ester.

Examples of $R^0$ being a precursor to a group R is when $R^0$ is a $C_{1-4}$alkylthio or $C_{1-4}$alkoxycarbonyl group.

Suitably a $C_{1-4}$alkylthio group can be converted to a $C_{1-4}$alkylsulphinyl group by treatment with one mole equivalent of an oxidising agent such as hydrogen peroxide or potassium periodate. A further mole equivalent of an oxidising agent such as hydrogen peroxide or potassium permanganate can be used to convert a $C_{1-4}$alkylsulphinyl group to a $C_{1-4}$alkylsulphonyl group.

A $C_{1-4}$alkoxycarbonyl group can be converted to a $CONR^9R^{10}$ group by reaction with an amine $HNR^9R^{10}$ wherein $R^9$ and $R^{10}$ are as hereinbefore defined.

A compound of the formula (1) wherein R is hydrogen can be converted to the corresponding compound wherein R is 5-nitro by reaction with a suitable nitrating agent, such as fuming nitric acid together with sulphuric acid.

A compound of the formula (1) wherein R is nitro can be converted to the corresponding compound wherein R is amino by reaction with a reducing agent, for example via catalytic hydrogenation with palladium on carbon. If desired the amino group can be converted to a $C_{1-4}$-alkanoylamino group by treatment with a $C_{1-4}$alkanoylating agent, for example acetic anhydride.

Alternatively a compound of the formula (1) wherein R is amino can be treated with sodium nitrite and an inorganic acid such as sulphuric acid to form a diazonium salt which can be converted to compounds of the formula (1) wherein R is cyano or halo by reaction with cuprous cyanide or cuprous halide.

A compound of the formula (1) wherein R is cyano can be hydrolysed to the corresponding compound wherein R is carboxamido by treatment with concentrated sulphuric acid or by treatment with hydrogen peroxide and potassium hydroxide.

A compound of the formula (1) wherein R is hydrogen can be converted to the corresponding compound wherein R is $5-SO_2NR^{13}R^{14}$ by reaction with chlorosulphonic acid and thereafter with an amine $HNR^{13}R^{14}$ wherein $R^{13}$ and $R^{14}$ are as hereinbefore defined.

As will be readily understood by the man skilled in the art, reactive groups in other parts of the molecule can be protected before the groups R and $R^2$ are converted as hereinbefore described.

Compounds of the formula (2) wherein Y is hydroxy can suitably be prepared by reaction under basic conditions of a compound of the formula (5) :

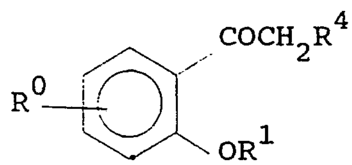

(5)

wherein R°, $R^1$ and $R^4$ are as hereinbefore defined,
with a compound of the formula $R^3COL$ wherein $R^3$ is as hereinbefore defined and L is a leaving group.

Suitably L is ethoxy or methoxy. Conveniently a solution of a compound of the formula (5) and a compound of the formula $R^3COL$ in a suitable organic solvent such as diethyl ether is treated with a suitable base such as an alkali metal alkoxide, e.g. sodium methoxide at ambient temperature. The resulting reaction mixture is preferably extracted with water and the aqueous extract with contains the alkali metal salt of a compound of the formula (2) wherein Y is hydroxy is then treated with a compound of the formula (3) as hereinbefore described.

Compounds of the formula (2) wherein Y is a secondary amino group (e.g. dimethylamino) can suitably be prepared by reacting a compound of the formula (5) with a compound of the formula $R^3C(OR)_2Y$ wherein $R^3$ is as hereinbefore defined, R is $C_{1-4}$alkyl and Y is a secondary amino group (for example $R^3C(OR)_2Y$ is N,N-dimethylformamide dimethyl or diethyl acetal).

A compound of the formula (4) can be prepared by reacting a compound of the formula (6) :

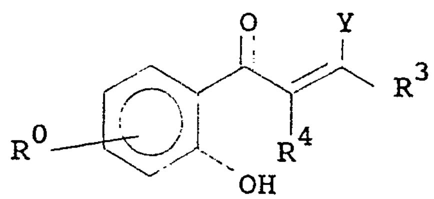

(6)

wherein Y, $R^0$, $R^3$ and $R^4$ are as hereinbefore defined, with a compound of the formula (3) as hereinbefore defined in a similar manner as hereinbefore described for the preparation of a compound of the formula (1).

Pharmaceutically acceptable base addition salts of the compounds of the formula (1) may be prepared by standard methods, for example by reacting a solution of the compound of the formula (1) with a solution of the base.

In another aspect this invention provides a compound of the formula (1) as hereinbefore defined and a pharmaceutically acceptable salt thereof, for use as a medicament.

In a further aspect this invention provides the use of a compound of the formula (7):

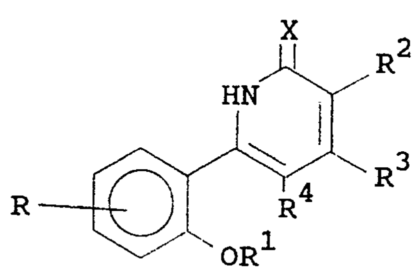

(7)

wherein X, R, $R^1$, $R^2$, $R^3$ and $R^4$ are as hereinbefore defined for a compound of the formula (1) except that $R^1$ can be methyl when $R^2$ is $-CO_2H$, $-CO_2CH_2CH_3$ or -CN, X is 0, $R^3$ is hydrogen, $R^4$ is hydrogen or methyl and R is 6-methoxy; in the manufacture of a medicament having bronchodilator and/or anti-allergic activity.

The compounds of the formula (7) can be prepared, formulated as pharmaceutical compositions and used as medicaments in methods of therapy as hereinbefore described for the compounds of formula (1).

The following biological test methods, data and Examples serve to illustrate this invention.

**Bronchodilatation - In vivo**

Male guinea-pigs of the Dunkin Hartley strain (500 - 600g) were anaesthetised with Sagatal (pentobarbital sodium) (60 mg/kg). Airway resistance was measured using a modification of the classical Konzett-Rossler technique (J. Pharm. Methods, 13, 309-315, 1985). U46619 (9,11-methanoepoxy-$PGH_2$)was infused i.v. at a rate of 2.5 nmol/min, this produced a steady state of bronchoconstriction (approximately

120% increase from basal airway resistance). The compound under test was administered by i.v. bolus injection, and the subsequent peak inhibition of bronchoconstriction recorded.

The dose of compound required to reduce the U46619-induced bronchoconstriction by 50% is given as the $BD_{50}$. The compounds of Examples 2(a), 7, 8, 9(b), 11, 13, 19, 23(b), 24 and 25 had $BD_{50}$ values in the range 0.29 to 9.1 µmol/kg. These results demonstrate in vivo anti-bronchoconstrictor activity.

## Anti-allergic activity

Male Duncan Hartley guinea-pigs (250-300 g) were sensitised to ovalbumen by i.p. injection of 2 ml of $50 mg.ml^{-1}$ i.p. and 0.2 ml s.c. Three weeks later they were anaesthetised with $60 mg.kg^{-1}$ sodium pentabarbitone. The trachea was cannulated and the animal respired at a rate of 40 breaths per minute and at an initial tracheal inflation pressure of 16 mmHg. Tracheal inflation pressure was measured by a transducer connected to a side arm of the respiration circuit. The carotid artery was cannulated for the measurement of blood pressure and the signal was used to trigger an instantaneous rate meter. A jugular vein was cannulated for the administration of drug and allergen. After surgery the animals were allowed to stabilise and the drug was administered i.v. as a bolus. Following this, ovalbumen $1 mg.kg^{-1}$ was injected i.v. as the antigen challenge either 2, 15 or 30 minutes following drug treatment and the peak bronchoconstrictor response recorded. For the control group ovalbumen only was given. One ovalbumen challenge per guinea-pig was used and n = 6 for each time point. The percentage increase in tracheal inflation pressure was calculated.

The compound of Example 25 at a dose of 17 µmol/kg caused a 40% inhibition of the control bronchoconstrictor response 30 minutes following drug administration indicating an anti-allergic activity.

## Phosphodiesterase activity

The activity of the compounds of the present invention as inhibitors of a calmodulin insensitive cyclic GMP phosphodiesterase was measured using the procedure described in European Patent Application Publication No. 293063. The compounds of Examples 2(a), 2(b), 4, 5, 7 to 9 and 21 to 25 had $IC_{50}$ values (the concentration of inhibitor required for 50% inhibition of enzyme activity) in the range 0.4 to 25 µM. The compounds of the present invention have the advantage that they are selective in not inhibiting cyclic AMP phosphodiesterase (type III).

## Inhibition of spontaneous colonic activity - in vitro

Male albino guinea-pigs (300 - 400g) were killed by a blow to the back of the head and exsanguinated. A 2cm long segment of the proximal part of the hypogastric loop of the distal colon was rapidly dissected out and placed in oxygenated (95% $O_2$, 5% $CO_2$) modified warm Krebs solution. The tissue was cleaned out with Krebs and the adjoining mesentery discarded. Cotton was then tied to each end and the colon was attached to a tissue holder in an organ bath containing modified oxygenated Krebs solution at 37°C. The other end of the tissue was tied to an isometric transducer and placed under 1g tension. Force developed by the muscle was detected by the transducer, and recorded on a multitrace pen recorder. Spontaneous colonic activity, as assessed by the contraction distance over a five minute period, was subjected to computer analysis.

The tissues were allowed to settle at a resting tension of 1g for 1 hour, during which time they were washed at 15 minute intervals. Three samples of pre-dose activity were taken and averaged. The tissues were then dosed and two samples of post-dose activity were taken. The lowest value was used to calculate the percentage relaxation, and log dose response curves were constructed. The tissues were washed 10 minutes after dosing and left for 15 minutes to settle prior to the next control period.

The concentration of compound required to reduce spontaneous colonic activity by 50% is given as the $IC_{50}$.

The compounds of Examples 9(b) and 13 had $IC_{50}$ values of 3 and 1.1 µM respectively.

## Example 1

3-Cyano-6-(2-methoxy-4-methylthiophenyl)-2(1H)-pyridinone

A stirred mixture of 2-methoxy-4-methylthioacetophenone (7 g), dimethylformamide dimethyl acetal (5.9 ml) and dimethylformamide (21 ml) was heated under reflux for 16 hours then cyanoacetamide (4.5 g) was added and heating was continued for a further 12 hours. The mixture was diluted with water (100 ml), acidified with hydrochloric acid, and the resultant solid was digested with ethanol to give a crude product, 3.94 g. Purification by chromatography (silica, dichloromethane and dichloromethanemethanol mixtures) followed by recrystallisation from acetonitrile yielded the pure title compound, 2.34 g, m.p. 245-247 °C.

## Example (2a) and (2b)

2(a) 3-Cyano-6-(4-methylthio-2-propoxyphenyl)-2(1H)-pyridinone

2(b) 1,2-Dihydro-6-(4-methylthio-2-propoxyphenyl)-2-oxo-3-pyridine carboxamide

In a similar manner to that of Example 1, 4-methylthio-2-propoxyacetophenone (9.35 g) yielded a mixture which was separated to give 3-cyano-6-(4-methylthio-2-propoxyphenyl)-2(1H)-pyridinone, 3.24 g, m.p. 196-197 °C (from acetonitrile), and 1,2-dihydro-6-(4-methylthio-2-propoxyphenyl)-2-oxo-3-pyridine carboxamide, 0.25 g, m.p. 254-255 °C (from aqueous ethanol).

The starting material was prepared by the following method :

1-Bromopropane (18.19 ml) was added at room temperature to a stirred solution of the sodium salt from 0-(4-acetyl-3-hydroxyphenyl)N,N-dimethylcarbamothioate (16 g) and sodium hydride (50%, 3.65 g) in dimethylformamide (180 ml). After 24 hours the mixture was diluted with water and extracted with ethyl acetate. The extract was washed with water and dilute sodium hydroxide. The residue left after evaporation was washed with water and petroleum ether to give 0-(4-acetyl-3-methoxyphenyl)-N,N-dimethylcarbamothioate, which had m.p. 86-88 °C after trituration which hot aqueous ethanol. The above intermediate (18.35 g) was heated at 230-240 °C for 2 hours. A solution of the resultant solid in methanol (100 ml) and 5 normal sodium hydroxide solution (64 ml) was heated under reflux for 3 hours and then diluted with ethyl acetate. The mixture was extracted with dilute hydrochloric acid and evaporation of the organic layer gave 4-thio-2-propoxyacetophenone, 9.5 g, m.p. 80-82 °C. Recrystallisation from petroleum ether gave analytical material m.p. 83-85 °C.

Methyl iodide (7.73 ml) was added to a solution of the above intermediate (9.0 g) and potassium hydroxide (7.18 g) in methanol (86 ml). After 30 minutes the mixture was diluted with ethyl acetate and washed with water. Evaporation gave 4-methylthio-2-propoxyacetophenone, 9.32 g, m.p. 55-57 °C after recrystallisation from petroleum ether.

## Example 3

3-Cyano-6-(2-methoxy-4-methylsulphinylphenyl)-2(1H)-pyridinone

A solution of 3-cyano-6-(2-methoxy-4-methylthiophenyl)-2(1H)-pyridinone (1.24 g) in dichloromethane (500 ml) was treated with m-chloroperbenzoic acid (85%, 0.92 g). After 2 hours the solution was washed with sodium bicarbonate solution and evaporated to give the crude product (1.27 g, m.p. 239-241 °C). purification by chromatography (silica, dichloromethane) followed by recrystallisation from aqueous ethanol yielded the pure title compound, 0.81 g, m.p. 243-245 °C.

## Example 4

**3-Cyano-6-(4-methylsulphinyl-2-propoxyphenyl)-2(1H)-pyridinone**

In a manner similar to that of Example 3, 3-cyano-6-(4-methylthio-2-propoxyphenyl)-2(1H)-pyridinone (0.98 g) yielded the title compound, 0.83 g, m.p. 183-184 °C (from acetonitrile).

**Example 5**

**3-Cyano-6-(4-methylsulphonyl-2-propoxyphenyl)-2(1H)-pyridinone**

The product of Example 3 (0.66 g) in dichloromethane (50 ml) was treated with m-chloroperbenzoic acid (0.42 g) at room temperature. After 3 hours the solution was washed with sodium bicarbonate and the residue left after evaporation was triturated with water to give a solid, 0.61 g. Recrystallisation from acetonitrile yielded the pure title compound, 0.2 g, m.p. 241-242 °C.

**Example 6**

**3-cyano-6-(2-methoxy-4-methylsulphonylphenyl)-2(1H)-pyridinone**

In a manner similar to that of Example 3 but using an excess of m-chloroperbenzoic acid, 3-cyano-6-(2-methoxy-4-methylthiophenyl)-2(1H)-pyridinone (0.65 g) gave the title compound, 6.34 g, m.p. 280-282 °C (from acetonitrile).

**Example 7**

**3-Cyano-6-(5-fluoro-2-propoxyphenyl)-2(1H)-pyridinone**

A stirred mixture of 3-dimethylamino-1-(5-fluoro-2-propxyphenyl)-2-propene-1-one (2.46 g), cyanoacetamide (0.93 g), sodium methoxide (from 50% sodium hydride, 1.69 g, and methanol, 0.85 ml) in dimethylformamide (20 ml) was heated under reflux for 7 hours. The mixture was poured into water (150 ml) and acetic acid added to pH 4 to give a crude product. Recrystallisation twice from acetonitrile gave a solid, 0.68 g, which was washed with petroleum ether and recrystallised again from acetonitrile to give the pure title compound, 0.15 g, m.p. 242-243 °C.

The starting-material was prepared by the following method :

A stirred mixture of 5-fluoro-2-hydroxyacetophenone (10 g), potassium iodide (0.75 g) potassium carbonate (10.5 g), 1-bromopropane (6 ml) and acetone (100 ml) was heated under reflux for 30 hours. The filtered solution was evaporated and the residue was dissolved in dichloromethane and washed with dilute sodium hydroxide solution. Evaporation of the organic solution gave a crude product, Kugelruhr distillation (1 mm, 230-280 °C) of which gave 5-fluoro-2-propoxyacetophenone, 8.83 g, m.p. 58-60 °C.

The above acetophenone (3.5 g) in dimethylformamide (20 ml) was heated under reflux with dimethylformamide dimethyl acetal (2.86 ml) for 48 hours. Additional dimethylformamide dimethyl acetal (1.5 ml) was added and the mixture was heated for a further 27 hours. After dilution with ethyl acetate (60 ml) the solution was washed with water, dried and evaporated. The residue was allowed to stand in the cold to give a solid which was triturated with petroleum ether to yield crude 3-dimethylamino-1-(5-fluoro-2-propoxyphenyl)-2-propene-1-one, 1.17 g, m.p. 43-46 °C. Further material (1.9 g) was recovered from the filtrate.

**Example 8**

**1,2-Dihydro-6-(5-fluoro-2-propoxyphenyl)-2-oxo-3-pyridine carboxamide**

The product of Example 7 (1.36 g) in a solution of potassium hydroxide (0.67 g) in water (15 ml) at 55 °C was treated with 30% hydrogen peroxide (6.5 ml) in portions during 4 hours. Additional potassium hydroxide solution was also added as required to maintain an alkaline solution. Acetic acid was added to pH 3 and the crude product was purified by flash chromatography (silica, chloroform) to yield 1.16 g of a solid m.p. 223-224 °C. Recrystallisation from aqueous ethanol gave the title compound, 1.04 g, m.p. 224-225 °C.

**Example (9a) and (9b)**

**9(a) 3-Cyano-6-(4-methoxy-2-propoxyphenyl)-2(1H)-pyridinone**

**9(b) 1,2-Dihydro-6-(4-methoxy-2-propoxyphenyl)-2-oxo-3-pyridine carboxamide**

In a similar manner to that of Example 1, 4-methoxy-2-propoxyacetophenone (7 g) yielded a mixture which was separated by flash chromatography to give 3-cyano-6-(4-methoxy-2-propoxyphenyl)-2(1H)-pyridinone, 2.6 g, m.p. 183-183.5 °C (from acetonitrile) and 1,2-dihydro-6-(4-methoxy-2-propoxyphenyl)-2-oxo-3-pyridine carboxamide, 0.22 g, m.p. 264-266 °C (from acetonitrile).

The starting material was prepared as an oil from 2-hydroxy-4-methoxyacetophenone by a similar method to that described in Example 7.

**Example 10**

**3-Cyano-6-(5-methoxy-2-propoxyphenyl)-2(1H)-pyridinone**

In a similar manner to that of Example 1, 5-methoxy-2-propoxyacetophenone (7.7 g) gave 3.06 g of a crude product, 1.4 g of which was purified by flash chromatography to give the title compound, 0.9 g, m.p. 214-215 °C (from acetonitrile).

The starting material was prepared as a solid (m.p. 41-43 °C) from 2-hydroxy-5-methoxyacetophenone by a similar method to that described in Example 7.

**Example 11**

**1,2-Dihydro-6-(5-methoxy-2-propoxyphenyl)-2-oxo-3-pyridine carboxamide**

In a similar manner to that of Example 8, 1.42 g of the crude product of Example 10 gave the title compound, 1.31 g, m.p. 199-201 °C (from acetonitrile).

**Example 12**

**3-Cyano-6-(5-cyano-2-propoxyphenyl)-2(1H)-pyridinone**

In a similar manner to that of Example 1, 5-cyano-2-propoxyacetophenone (8.53 g) gave a reaction mixture which was poured into water (250 ml) and dichloromethane (350 ml). Filtration gave 1.62 g of a solid m.p. 248-251 °C. The residue left after evaporation of the organic solution was digested with ether

(3x100 ml) and the digest allowed to stand in the cold gave a further 1.45 g of solid m.p. 243-246 °C. A portion of the crude product (1 g) was recrystallised from aqueous acetonitrile and then from acetonitrile to give the title compound, 0.65 g, m.p. 254-255 °C.

The starting material was prepared as a solid m.p. 122-123 °C (from aqueous acetonitrile) from 5-cyano-2-hydroxyacetophenone by a similar method to that described in Example 7.

## Example 13

### 3-(3-Carboxamido-1,2-dihydro-2-oxo-6-pyidinyl)-4-propoxybenzamide

A stirred suspension of the product of Example 12 (0.66 g) in benzene (75 ml) was heated under reflux with manganese dioxide on silica (11.32 g, Synthesis, 1988, 715-717) for 18 hours. The cool mixture was filtered and the solid was digested twice with a hot mixture of methanol (100 ml) and concentrated aqueous ammonia (15 ml). Evaporation of the digest left a residue which was triturated with water to give 0.66 g of a solid, m.p 275-277 °C. This was combined with a further 0.2 g made in a similar manner and recrystallised twice from dimethylformamide to give the title compound, 0.45 g, m.p. 279-281 °C.

## Example 14

### Methyl-3-(3-cyano-1,2-dihydro-(2-oxo-6-pyridinyl)-4-propoxybenzoate

In a similar manner to that of Example 1, methyl 3-acetyl-4-propoxybenzoate (4.3 g) gave 1.1 g of a solid m.p. 201-203 °C. Recrystallisation from acetonitrile gave the title compound, 0.86 g, m.p. 203-204 °C.

The starting material was prepared as follows :

A mixture of 5-cyano-2-propoxyacetophenone (9.6 g, prepared as in Example 12), acetic acid (90 ml) and concentrated hydrochloric acid (90 ml) was heated under reflux for 18 hours. The mixture was evaporated and the residue triturated with water to give 3-acetyl-4-propoxybenzoic acid, 9.7 g, m.p. 165-169 °C.

The above acid (9.6 g) was esterified with methanol/sulphuric acid and the crude product was purified by flash chromatography to give methyl 3-acetyl-4-propoxybenzoate, 8.17 g, m.p. 44-47 °C.

## Example 15

### 3-(3-cyano-1,2-dihydro-2-oxo-6-pyridinyl)-4-propxoybenzamide

A mixture of the product of Example 14 (1 g) and saturated methanolic ammonia (50 ml) was heated in a pressure vessel at 100 °C for 16 hours. Evaporation gave a crude product which after flash chromatography and recrystallisation (aqueous ethanol) gave 0.22 g of the title compound contaminated with some unreacted starting material. This solid was combined with a further 0.28 g, made in a similar manner, and flash chromatography (silica, gradient elution using 5% methanol in chloroform and 1-5% ammonia) gave the title compound, 0.31 g, m.p. 246-247 °C (from aqueous ethanol).

## Example 16

### N-Methyl-3-(3-cyano-1,2-dihydro-2-oxo-6-pyridinyl)-4-propoxybenzamide

A mixture of methyl 3-(3-cyano-1,2-dihydro-2-oxo-6-pyridinyl)-4-propoxybenzoate (2.0 g) and 1 Normal sodium hydroxide (30 ml) was heated under reflux for 45 minutes and the resultant solution was acidified to give a quantitative yield of 3-(3-cyano-1,2-dihydro-2-oxo-6-pyridinyl)-4-propoxybenzoic acid, m.p. 277-279 °C. This acid was heated with thionyl chloride (10 ml) for 90 minutes, the solution was evaporated, and the acid chloride was dissolved in dichloromethane (60 ml). Methylamine was passed slowly through the stirred solution for 45 minutes and both the resultant solid and solution were washed with dilute hydrochloric acid and water. The solid was combined with the residue left after evaporation of the solution to give 1.66 g of solid m.p. 234-236 °C. Recrystallisation from ethanol gave the title compound, m.p. 235-237 °C.

The starting material was prepared as follows :

A stirred mixture of 5-cyano-2-propoxyacetophenone (20.3 g), glacial acetic acid (150 ml) and concentrated hydrochloric acid (70 ml) was heated under reflux for 16 hours. The chilled mixture was then filtered and the collected solid was washed with water, then dissolved in dilute aqueous sodium hydroxide solution. Acidification of the filtered solution gave 3-acetyl-4-propoxybenzoic acid, 15.57 g, m.p. 177-179 °C.

A stirred mixture of the above intermediate (14.34 g), methanol (200 ml) and sulphuric acid (2.0 ml) was heated under reflux for 20 hours. The residue after evaporation was distributed between water (150 ml) and dichloromethane (150 ml) and the organic layer was washed with water then aqueous potassium bicarbonate. The residue left after evaporation of the dried solution was triturated with petroleum ether to give methyl 3-acetyl-4-propoxybenzoate, 13.6 g, m.p. 55-56 °C.

In a similar manner to that of Example 1, the above intermediate (13.5 g) gave 4.77 g of a solid which was purified to give methyl 3-(3-cyano-1,2-dihydro-2-oxo-6-pyridinyl)-4-propoxybenzoate, 4.14 g, m.p. 201-203 °C.

## Example 17

**N-Methyl-3-(3-carboxamido-1,2-dihydro-2-oxo-6-pyridinyl)-4-propoxybenzamide**

In a similar manner to that of Example 13, N-methyl-3-(3-cyano-1,2-dihydro-2-oxo-6-pyridinyl)4-propoxybenzamide (0.45 g) gave 0.38 g of a crude product. Purification by flash chromatography and recrystallisation from acetonitrile/methanol gave the title compound, 0.2 g, m.p. 286-288 °C.

## Example 18

**N,N-Dimethyl-3-(3-cyano-1,2-dihydro-2-oxo-6-pyridinyl)-4-propoxybenzamide**

In a similar method to that of Example 16, methyl 3-(3-cyano-1,2-dihydro-2-oxo-6-pyridinyl)-4-propoxybenzoate (2.14 g) was saponified, converted into the acid chloride and treated with dimethylamine to give 2.0 g of a solid. This was combined with a further 0.2 g obtained in a similar manner, and purified by flash chromatography to give 1.9 g of solid m.p. 201-202 °C. Recrystallisation of part of this gave the title compound, m.p. 214-215 °C.

## Example 19

**N,N-dimethyl-3-(3-carboxamido-1,2-dihydro-2-oxo-6-pyridinyl)-4-propoxybenzamide**

In a similar manner to that of Example 8, the product of Example 18 (1.1 g) gave 0.79 g of a solid which was purified by flash chromatography and recrystallisation from acetonitrile/ethanol to give the title compound, 0.4 g, m.p. 203-204 °C.

## Example 20

### 4-(3-Cyano-1,2-dihydro-2-oxo-6-pyridinyl)-3-propoxybenzonitrile

In a similar manner to that of Example 1, 4-acetyl-3-propoxybenzonitrile (3.6 g) gave 2.17 g of the title compound containing about 10 % of the corresponding amide. A portion of the crude product (0.55 g) was heated under reflux with phosphoryl chloride (10 ml) for 90 minutes and the resultant solution was evaporated. The residue was treated with ice/water (50 ml) and the mixture was then stirred at room temperature for 30 minutes, neutralised with potassium bicarbonate, and filtered to give 0.48 g of a solid. Recrystallisation twice from dimethylformamide gave the title compound, 0.2 g, m.p. 304-306° C.

The starting material was prepared as follows :

In a manner similar to that given in Example 7, 2-hydroxy-4-iodoacetophenone (9.27 g) gave 4-iodo-2-propoxyacetophenone, 6.97 g, m.p. 97.5-98.5° C.

A stirred mixture of the above intermediate (6.79 g) and cuprous cyanide (6.0 g) in 1,3-dimethyl-2-imidazolidinone (50 ml) was heated at 120° C for 4 hours, then cooled and poured into a stirred mixture of aqueous potassium cyanide (21.78 g in water, 150 ml) and dichloromethane (200 ml). After 2 hours the organic layer was washed with water, dried, and evaporated to give 3.94 g of a solid. Purification by flash chromatography gave 4-acetyl-3-propoxybenzonitrile, 3.71 g, m.p. 99.5-100° C.

## Example 21

### 4-(3-Carboxamido-1,2-dihydro-2-oxo-6-pyridinyl)-3-propoxybenzamide

In a similar manner to that of Example 13, crude 4-(3-cyano-1,2-dihydro-2-oxo-6-pyridinyl)-3-propoxybenzonitrile (0.33 g) gave 0.33 g of a solid which was recrystallised from dimethylformamide to give the title compound, 0.22 g, m.p. 296-298° C.

## Example 22

### 3-Cyano-6-(5-methylthio-2-propoxyphenyl)-2(1H)pyridinone

In a manner similar to that of Example 1, 5-methylthio-2-propoxyacetophenone (7.75 g) gave 3.23 g of a solid, which was recrystallised in turn from acetonitrile, ethanol, and dimethylformamide to give the title compound, 0.26 g, m.p. 204-205° C.

The starting material was prepared as follows :

A solution of 3-acetyl-4-hydroxyphenyldiazonium chloride, prepared from 5-amino-2-hydroxyacetophenone (11.9 g) and hydrochloric acid (13.5 ml) in water (80 ml) with sodium nitrite (5.59 g) in water (15 ml), was added dropwise during 45 minutes to a stirred solution of potassium xanthate (17.7 g) in water (40 ml) at 70-75° C, then the temperature was raised to 90° C for 30 minutes. Sodium hydroxide (10.0 g) was added and the stirred mixture was heated under reflux in an inert atmosphere for 18 hours. The cool mixture was acidified with 50% sulphuric acid, extracted with chloroform, and the combined extracts were washed with water and sodium bicarbonate solution. The residue left after evaporation was triturated with ether and some disulphide (2.88 g) was removed by filtration. Evaporation of the filtrate gave 7.86 g of a solid which was combined with a further 1.76 g of crude product obtained by stannous chloride reduction of the disulphide. Kugelruhr distillation (120-130° C, 1.0-0.7 mm Hg) gave 2-hydroxy-5-thioacetophenone, 7.15 g, m.p. 50-52° C.

Iodomethane (2.6 ml) was added dropwise to a stirred partial solution of the sodium salt prepared from the above intermediate (7.1 g) and 50% sodium hydride in oil (2.03 g) in dimethylformamide (35 ml). The resultant mixture was poured into water, hydrochloric acid added to pH 4, and the mixture was extracted with chloroform (4x50 ml). Evaporation of the washed and dried extract gave 7.95 g of an oil, flash

chromatography of which gave 2-hydroxy-5-methylthioacetophenone as a pale yellow oil, 6.05 g.

In a manner similar to that given in Example 7, the above intermediate (6.75 g) gave 5-methylthio-2-propoxyacetophenone, 7.9 g, as a pale orange coloured oil.

## Example 23(a) and 23(b)

### 23(a) 3-(3-cyano-1,2-dihydro-2-oxo-6-pyridinyl) 4-propoxy-N,N-dimethylbenzenesulphonamide

### 23(b) 3-(3-Carboxamido-1,2-dihydro-2-oxo-6-pyridinyl)-4-propoxy-N,N-dimethylbenzenesulphonamide

In a similar manner to that of Example 1, 3-acetyl-4-propoxy-N,N-dimethylbenzenesulphonamide (10.6 g) yielded a mixture which was separated to give 3-(3-cyano-1,2-dihydro-2-oxo-6-pyridinyl)-4-propoxy-N,N-dimethylbenzenesulphonamide, 3.74 g, m.p. 225-226°C (from acetonitrile), and 3-(3-carboxamido-1,2-dihydro-2-oxo-6-pyridinyl)-4-propoxy-N,N-dimethylbenzenesulphonamide, 1,2 g, m.p. 250-252°C (from aqueous ethanol).

The starting material was prepared by the following method :

A vigorously stirred mixture of 3-acetyl-4-hydroxybenzene sulphonic acid ammonium salt (29.5 g) and phosphoryl chloride (150 ml) was heated under reflux for 2 hours. The residue left after evaporation was very carefully added to 33 % dimethylamine in ethanol (200 ml) cooled to 0°C. After the vigorous reaction had subsided the mixture was stirred for 2 hours at room temperature, allowed to stand overnight, and then evaporated. A solution of the residue in dichloromethane was washed with dilute hydrochloric acid and then extracted with 1 Normal sodium hydroxide solution (3x75 ml). Acidification of the extract gave a sticky solid which was purified by flash chromatography to yield 3-acetyl-4-hydroxy-N,N-dimethylbenzenesulphonamide,14.0 g, m.p. 115-116°C (from cyclohexane/toluene).

In a similar manner to that given in Example 7, the above intermediate (10.0 g) gave 3-acetyl-4-propoxy-N,N-dimethylbenzenesulphonamide, 10.94 g, m.p. 91.5-92°C (from cyclohexane/toluene).

## Example 24

### 6-(2-Cyclopropylmethoxy-5-flourophenyl)-1,2-dihydro-2-oxo-pyridine-3-carboxamide

In a similar manner to that of Example 8, 3-cyano-6-(2-cyclopropylmethoxy-5-fluorophenyl)2(1H)-pyridinone (1.72 g) yielded the title compound, 1.08 g, m.p. 169-170°C (from acetonitrile).

The starting material was prepared by the following method :

In a similar manner to that given in Example 7, but using cyclopropylmethylbromide, 5-fluoro-2-hydroxyacetophenone (8.5 g) yielded 2-cyclopropylmethoxy-5-fluoroacetophenone, 7.9 g, as a pale orange oil.

In a manner similar to that of Example 1, the above intermediate (7.8 g) yielded 3-cyano-6-(2-cyclopropylmethyl-5-fluorophenyl)-2(1H)-pyridinone, 3.05 g, m.p. 238-240°C.

## Example 25

### 6-(5-Fluoro-2-(2-methylpropoxy)phenyl)-1,2-dihydro-2-oxo-pyridine-3-carboxamide

In a similar manner to that of Example 8, 3-cyano-6-(5-fluoro-2-(2-methylpropoxy)phenyl)-2(1H)-pyridinone (1.88 g) yielded the title compound, 1.05 g, m.p. 193-194°C (from acetonitrile).

The starting material was prepared by the following method :

In a similar manner to that given in Example 7, but using 1-bromo-2-methylpropane, 5-fluoro-2-hydroxyacetophenone (8.15 g) yielded 5-fluoro-2-(2-methylpropoxy)acetophenone, 5.1 g, as a pale brown semisolid.

In a manner similar to that of Example 1, the above intermediate (7.0 g) yielded 3-cyano-6-(5-fluoro-2-(2-methylpropoxy)phenyl)-2(1H)-pyridinone, 2.28 g, m.p. 50-52° C.

## Example 26

Pharmaceutical compositions for oral administration are prepared by combining the following :

| | % w/w | | |
|---|---|---|---|
| 3-(3-Cyano-1,2-dihydro-2-oxo-6-pyridinyl)-4-propxoybenzamide | 0.5 | 3.0 | 7.14 |
| 2% w/w Soya lecithin in soya bean oil | 90.45 | 88.2 | 84.41 |
| Hydrogenated vegetable shortening and beeswax | 9.05 | 8.8 | 8.45 |

The formulations are then filled into individual soft gelatin capsules.

## Example 27

A pharmaceutical composition for parenteral administration is prepared by dissolving the title compound of Example 9(b) (0.02 g) in polyethylene glycol 300 (25 ml) with heating. This solution is then diluted with water for injections Ph. Eur. (to 100 ml). The solution is then sterilised by filtration through a 0.22 micron membrane filter and sealed in sterile containers.

## Claims

1. A compound of the formula (1) :

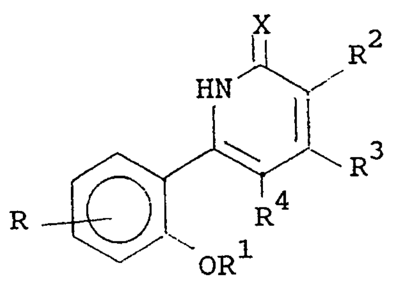

(1)

or a pharmaceutically acceptable salt thereof, wherein
X is O or S;
$R^1$ is $C_{1-6}$alkyl, $C_{2-6}$alkenyl, $C_{3-5}$cycloalkyl$C_{1-4}$alkyl, or $C_{1-4}$alkyl substituted by 1 to 3 fluoro groups;

$R^2$ is hydrogen, -CN, $-CONR^5R^6$, $-CO_2R^7$,5-tetrazolyl, $-NO_2$, $-NH_2$ or $-NHCOR^8$ wherein $R^5$ to $R^8$ are independently hydrogen or $C_{1-4}$alkyl;
$R^3$ is hydrogen or $C_{1-4}$alkyl;
$R^4$ is hydrogen or $C_{1-4}$alkyl; and
R is halo, $C_{1-4}$alkyl, $C_{1-4}$alkoxy, cyano, $-CONR^9R^{10}$, $-CO_2R^{11}$, $-S(0)_nC_{1-4}$alkyl, $-NO_2$, $-NH_2$, $-NHCOR^{12}$, or $-SO_2NR^{13}R^{14}$ wherein n is 0, 1 or 2 and $R^9$ to $R^{14}$ are independently hydrogen or $C_{1-4}$alkyl;
with the proviso that $R^1$ is not methyl when $R^2$ is $-CO_2H$, $-CO_2CH_2CH_3$ or -CN, X is 0, $R^3$ is hydrogen, $R^4$ is hydrogen or methyl and R is 6-methoxy.

2. A compound according to claim 1 wherein X is O.
3. A compound according to claim 1 or 2 wherein $R^1$ is $C_{2-6}$alkyl.
4. A compound according to claim 1 or 2 wherein $R^1$ is $C_{3-5}$alkenyl.
5. A compound according to any one of claims 1 to 4 wherein $R^2$ is hydrogen, -CN, 5-tetrazolyl or $-NO_2$.
6. A compound according to any one of claims 1 to 4 wherein $R^2$ is $-CONR^5R^6$ wherein $-NR^5R^6$ is amino.
7. A compound according to any one of claims 1 to 4 wherein $R^2$ is $-CO_2R^7$ wherein $R^7$ is methyl.
8. A compound according to any one of claims 1 to 7 wherein $R^3$ is hydrogen or methyl.
9. A compound according to any one of claims 1 to 8 wherein $R^4$ is hydrogen or methyl.
10. A compound according to any one of claims 1 to 9 wherein R is halo, $C_{1-4}$alkyl or $C_{1-4}$alkoxy.
11. A compound according to any one of claims 1 to 9 wherein R is cyano, $-CONR^9R^{10}$ or $-CO_2R^{11}$ wherein $R^{11}$ is $C_{1-4}$alkyl.
12. A compound according to any one of claims 1 to 9 wherein R is $-NO_2$, $-NH_2$ or $-NHCOR^{12}$ wherein $R^{12}$ is $C_{1-4}$alkyl.
13. A compound according to any one of claims 1 to 9 wherein R is $-S(0)_nC_{1-4}$alkyl or $-SO_2NR^{13}R^{14}$.
14. A compound according to claim 1 which is :
3-cyano-6-(2-methoxy-4-methylthiophenyl)-2(1H)-pyridinone,
3-cyano-6-(4-methylthio-2-propoxyphenyl)-2(1H)-pyridinone,
1,2-dihydro-6-(4-methylthio-2-propoxyphenyl)-2-oxo-3-pyridine carboxamide,
3-cyano-6-(2-methoxy-4-methylsulphinylphenyl)-2(1H)-pyridinone,
3-cyano-6-(4-methylsulphinyl-2-propoxyphenyl)-2(1H)-pyridinone,
3-cyano-6-(4-methylsulphonyl-2-propoxyphenyl)-2(1H)-pyridinone,
3-cyano-6-(2-methoxy-4-methylsulphonylphenyl)-2(1H)-pyridinone,
3-cyano-6-(5-fluoro-2-propoxyphenyl)-2(1H)-pyridinone,
1,2-dihydro-6-(5-fluoro-2-propoxyphenyl)-2-oxo-3-pyridine carboxamide,
3-cyano-6-(4-methoxy-2-propoxyphenyl)-2(1H)-pyridinone,
1,2-dihydro-6-(4-methoxy-2-propoxyphenyl)-2-oxo-3-pyridine carboxamide,
3-cyano-6-(5-methoxy-2-propoxyphenyl)-2(1H)-pyridinone,
1,2-dihydro-6-(5-methoxy-2-propoxyphenyl)-2-oxo-3-pyridine carboxamide,
3-cyano-6-(5-cyano-2-propoxyphenyl)-2(1H)-pyridinone,
3-(3-carboxamido-1,2-dihydro-2-oxo-6-pyridinyl)-4-propoxybenzamide,
methyl 3-(3-cyano-1,2-dihydro-(2-oxo-6-pyridinyl)-4-propoxybenzoate,
3-(3-cyano-1,2-dihydro-2-oxo-6-pyridinyl)-4-propoxybenzamide,
N-methyl-3-(3-cyano-1,2-dihydro-2-oxo-6-pyridinyl)-4-propoxybenzamide,
N-methyl 3-(3-carboxamido-1,2-dihydro-2-oxo-6-pyridinyl)-4-propoxybenzamide,
N,N-dimethyl-3-(3-cyano-1,2-dihydro-2-oxo-6-pyridinyl)-4-propoxybenzamide,
N,N-dimethyl 3-(3-carboxamido-1,2-dihydro-2-oxo-6-pyridinyl)-4-propoxybenzamide,
4-(3-cyano-1,2-dihydro-2-oxo-6-pyridinyl)-3-propoxybenzonitrile,
4-(3-carboxamido-1,2-dihydro-2-oxo-6-pyridinyl)-3-propoxybenzamide,
3-cyano-6-(5-methylthio-2-propoxyphenyl)-2(1H)pyridinone,
3-(3-cyano-1,2-dihydro-2-oxo-6-pyridinyl)-4-propoxy-N,N-dimethylbenzenesulphonamide,
3-(3-carboxamido-1,2-dihydro-2-oxo-6-pyridinyl)-4-propoxy-N,N-dimethylbenzenesulphonamide,
6-(2-cyclopropylmethoxy-5-flourophenyl)-1,2-dihydro-2-oxopyridine-3-carboxamide,
6-(5-fluoro-2-(2-methylpropoxy)phenyl)-1,2-dihydro-2-oxopyridine-3-carboxamide,
3-cyano-6-(5-nitro-2-propoxyphenyl)-2(1H)-pyridinone,
1,2-dihydro-6-(5-nitro-2-propoxyphenyl)-2-oxo-3-pyridinone carboxamide,
3-cyano-6-(5-amino-2-propoxyphenyl)-2(1H)-pyridinone,
1,2-dihydro-6-(5-amino-2-propoxyphenyl)-2-oxo-3-pyridinone carboxamide,
3-cyano-6-(5-acetamido-2-propoxyphenyl)-2(1H)-pyridinone or
1,2-dihydro-6-(5-acetamido-2-propoxyphenyl)-2-oxo-3-pyridine carboxamide,
or a pharmaceutically acceptable salt thereof.

15. A compound according to any one of claims 1 to 14 for use as a medicament.

16. A pharmaceutical composition which comprises a compound according to any one of claims 1 to 15 and a pharmaceutically acceptable carrier.

17. A process for preparing a compound of the formula (1) or a pharmaceutically acceptable salt thereof as defined in claim 1 which process comprises :

a) reacting a compound of the formula (2) :

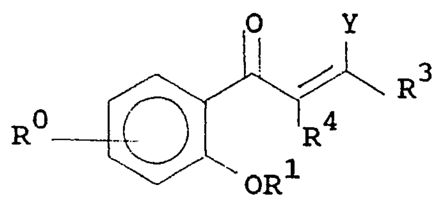

(2)

wherein $R^1$, $R^3$ and $R^4$ are as defined in claim 1, $R^0$ is a group R as defined in claim 1 or a precursor thereof and Y is a displaceable group,
with a compound of the formula (3) :

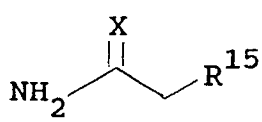

(3)

wherein X is as defined in claim 1 and $R^{15}$ is a group $R^2$ as defined in claim 1 or a precursor thereof;

b) reacting a compound of the formula (4) :

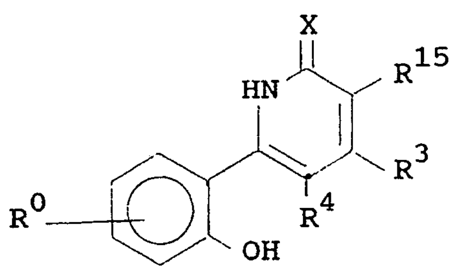

(4)

wherein $R^0$, X, $R^3$, $R^4$ and $R^{15}$ are as hereinbefore defined,
with a reagent that provides an $R^1$ group and thereafter where necessary :

° converting a group $R^{15}$ to a group $R^2$;

° converting a group $R^0$ to a group R;

° optionally forming a pharmaceutically acceptable salt.

18. A compound of the formula (2) as defined in claim 17.

19. A compound of the formula (4) as defined in claim 17.

20. The use of a compound of the formula (7):

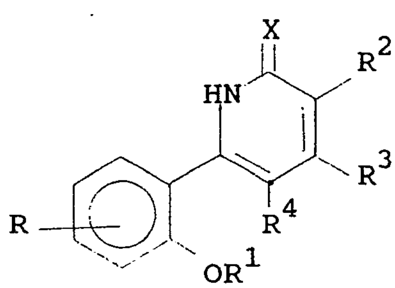

(7)

wherein X, R, $R^1$, $R^2$, $R^3$ and $R^4$ are as defined in claim 1 except that $R^1$ can be methyl when $R^2$ is $-CO_2H$, $-CO_2CH_2CH_3$ or -CN, X is 0, $R^3$ is hydrogen, $R^4$ is hydrogen or methyl and R is 6-methoxy; in the manufacture of a medicament having bronchodilator and/or anti-allergic activity.

Claims for the following Contracting States: ES, GR

1. A process for preparing a compound of the formula (1) :

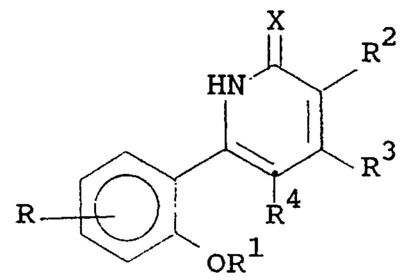

(1)

or a pharmaceutically acceptable salt thereof, wherein
X is O or S;
$R^1$ is $C_{1-6}$alkyl, $C_{2-6}$alkenyl, $C_{3-5}$cycloalkyl$C_{1-4}$alkyl, or $C_{1-4}$alkyl by 1 to 3 fluoro groups;
$R^2$ is hydrogen, -CN, $-CONR^5R^6$, $-CO_2R^7$,5-tetrazolyl, $-NO_2$, $-NH_2$ or $-NHCOR^8$ wherein $R^5$ to $R^8$ are independently hydrogen or $C_{1-4}$alkyl;
$R^3$ is hydrogen or $C_{1-4}$alkyl;
$R^4$ is hydrogen or $C_{1-4}$alkyl; and
R is halo, $C_{1-4}$alkyl, $C_{1-4}$alkoxy, cyano, $-CONR^9R^{10}$, $-CO_2R^{11}$, $-S(0)_nC_{1-4}$alkyl, $-NO_2$, $-NH_2$, $-NHCOR^{12}$, or $-SO_2NR^{13}R^{14}$ wherein n is 0, 1 or 2 and $R^9$ to $R^{14}$ are independently hydrogen or $C_{1-4}$alkyl;
with the proviso that $R^1$ is not methyl when $R^2$ is $-CO_2H$, $-CO_2CH_2CH_3$ or -CN, X is 0, $R^3$ is hydrogen, $R^4$ is hydrogen or methyl and R is 6-methoxy;
which process comprises:
a) reacting a compound of the formula (2) :

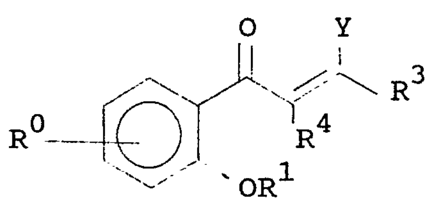

(2)

wherein $R^1$, $R^3$ and $R^4$ are as hereinbefore defined, $R^0$ is a group R as hereinbefore defined or a precursor thereof and Y is a displaceable group,
with a compound of the formula (3) :

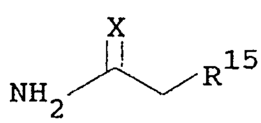

(3)

wherein X is as hereinbefore defined and $R^{15}$ is a group $R^2$ as hereinbefore defined or a precursor thereof;
b) reacting a compound of the formula (4) :

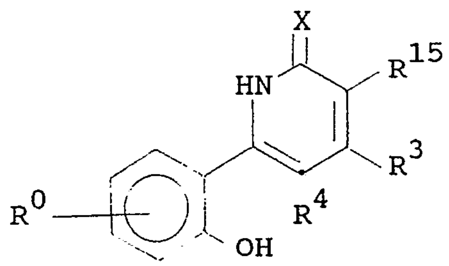

(4)

wherein $R^0$, X, $R^3$, $R^4$ and $R^{15}$ are as hereinbefore defined, with a reagent that provides an $R^1$ group and thereafter where necessary :

° converting a group $R^{15}$ to a group $R^2$;
° converting a group $R^0$ to a group R;
° optionally forming a pharmaceutically acceptable salt.

2. A process according to claim 1 for preparing a compound wherein X is O.
3. A process according to claim 1 or 2 for preparing a compound wherein $R^1$ is $C_{2-6}$alkyl.
4. A process according to claim 1 or 2 for preparing a compound wherein $R^1$ is $C_{3-5}$alkenyl.
5. A process according to any one of claims 1 to 4 for preparing a compound wherein $R^2$ is hydrogen, -CN, 5-tetrazolyl or $-NO_2$.
6. A process according to any one of claims 1 to 4 for preparing a compound wherein $R^2$ is $-CONR^5R^6$ wherein $-NR^5R^6$ is amino.
7. A process according to any one of claims 1 to 4 for preparing a compound wherein $R^2$ is $-CO_2R^7$ wherein $R^7$ is methyl.
8. A process according to any one of claims 1 to 7 for preparing a compound wherein $R^3$ is hydrogen or methyl.
9. A process according to any one of claims 1 to 8 for preparing a compound wherein $R^4$ is hydrogen or methyl.
10. A process according to any one of claims 1 to 9 for preparing a compound wherein R is halo, $C_{1-4}$alkyl or $C_{1-4}$alkoxy.
11. A process according to any one of claims 1 to 9 for preparing a compound wherein R is cyano, $-CONR^9R^{10}$, or $-CO_2R^{11}$ wherein $R^{11}$ is $C_{1-4}$alkyl.
12. A process according to any one of claims 1 to 9 for preparing a compound wherein R is $-NO_2$, $-NH_2$ or $-NHCOR^{12}$ wherein $R^{12}$ is $C_{1-4}$alkyl.
13. A process according to any one of claims 1 to 9 for preparing a compound wherein R is $-S(0)_nC_{1-4}$alkyl or $-SO_2NR^{13}R^{14}$.
14. A process according to claim 1 for preparing a compound which is :

3-cyano-6-(2-methoxy-4-methylthiophenyl)-2(1H)-pyridinone,
3-cyano-6-(4-methylthio-2-propoxyphenyl)-2(1H)-pyridinone,
1,2-dihydro-6-(4-methylthio-2-propoxyphenyl)-2-oxo-3-pyridine carboxamide,
3-cyano-6-(2-methoxy-4-methylsulphinylphenyl)-2(1H)-pyridinone,
3-cyano-6-(4-methylsulphinyl-2-propoxyphenyl)-2(1H)-pyridinone,
3-cyano-6-(4-methylsulphonyl-2-propoxyphenyl)-2(1H)-pyridinone,
3-cyano-6-(2-methoxy-4-methylsulphonylphenyl)-2(1H)-pyridinone,
3-cyano-6-(5-fluoro-2-propoxyphenyl)-2(1H)-pyridinone,
1,2-dihydro-6-(5-fluoro-2-propoxyphenyl)-2-oxo-3-pyridine carboxamide,
3-cyano-6-(4-methoxy-2-propoxyphenyl)-2(1H)-pyridinone,
1,2-dihydro-6-(4-methoxy-2-propoxyphenyl)-2-oxo-3-pyridine carboxamide,
3-cyano-6-(5-methoxy-2-propoxyphenyl)-2(1H)-pyridinone,
1,2-dihydro-6-(5-methoxy-2-propoxyphenyl)-2-oxo-3-pyridine carboxamide,
3-cyano-6-(5-cyano-2-propoxyphenyl)-2(1H)-pyridinone,
3-(3-carboxamido-1,2-dihydro-2-oxo-6-pyridinyl)-4-propoxybenzamide,
methyl 3-(3-cyano-1,2-dihydro-(2-oxo-6-pyridinyl)-4-propoxybenzoate,
3-(3-cyano-1,2-dihydro-2-oxo-6-pyridinyl)-4-propoxybenzamide,
N-methyl-3-(3-cyano-1,2-dihydro-2-oxo-6-pyridinyl)-4-propoxybenzamide,
N-methyl 3-(3-carboxamido-1,2-dihydro-2-oxo-6-pyridinyl)-4-propoxybenzamide,
N,N-dimethyl-3-(3-cyano-1,2-dihydro-2-oxo-6-pyridinyl)-4-propoxybenzamide,

N,N-dimethyl 3-(3-carboxamido-1,2-dihydro-2-oxo-6-pyridinyl)-4-propoxybenzamide,
4-(3-cyano-1,2-dihydro-2-oxo-6-pyridinyl)-3-propoxybenzonitrile,
4-(3-carboxamido-1,2-dihydro-2-oxo-6-pyridinyl)-3-propoxybenzamide,
3-cyano-6-(5-methylthio-2-propoxyphenyl)-2(1H)pyridinone,
3-(3-cyano-1,2-dihydro-2-oxo-6-pyridinyl)-4-propoxy-N,N-dimethylbenzenesulphonamide,
3-(3-carboxamido-1,2-dihydro-2-oxo-6-pyridinyl)-4-propoxy-N,N-dimethylbenzenesulphonamide,
6-(2-cyclopropylmethoxy-5-flourophenyl)-1,2-dihydro-2-oxopyridine-3-carboxamide
6-(5-fluoro-2-(2-methylpropoxy)phenyl)-1,2-dihydro-2-oxopyridine-3-carboxamide,
3-cyano-6-(5-nitro-2-propoxyphenyl)-2(1H)-pyridinone,
1,2-dihydro-6-(5-nitro-2-propoxyphenyl)-2-oxo-3-pyridinone carboxamide,
3-cyano-6-(5-amino-2-propoxyphenyl)-2(1H)-pyridinone,
1,2-dihydro-6-(5-amino-2-propoxyphenyl)-2-oxo-3-pyridinone carboxamide,
3-cyano-6-(5-acetamido-2-propoxyphenyl)-2(1H)-pyridinone or
1,2-dihydro-6-(5-acetamido-2-propoxyphenyl)-2-oxo-3-pyridine carboxamide,
or a pharmaceutically acceptable salt thereof.

15. A process according to claim 1 wherein Y is hydroxy or a derivative thereof or is a secondary amino group.

16. A process according to claim 1 wherein a reagent that provides an $R^1$ group is a compound of the formula $R^1Z$ wherein $R^1$ is as defined in claim 1 and Z is a leaving group.

17. A process according to claim 16 wherein Z is a halo, tosyl, mesyl or $OSO_3R^1$ group wherein $R^1$ is as defined in claim 16.

18. A process for preparing a pharmaceutical composition which comprises bringing into association a compound according to any one of claims 1 to 14 and a pharmaceutically acceptable carrier.

19. The use of a compound of the formula (7):

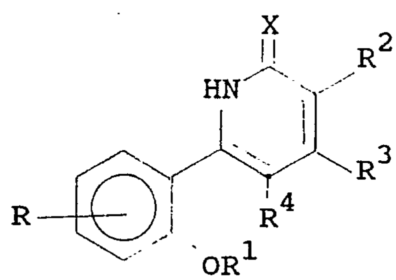

(7)

wherein X, R, $R^1$, $R^2$, $R^3$ and $R^4$ are as defined in claim 1 except that $R^1$ can be methyl when $R^2$ is $-CO_2H$, $-CO_2CH_2CH_3$ or -CN, X is 0, $R^3$ is hydrogen, $R^4$ is hydrogen or methyl and R is 6-methoxy; in the manufacture of a medicament having bronchodilator and/or anti-allergic activity.